## Europäisches Patentamt
## European Patent Office
## Office européen des brevets

(19)

(11) Veröffentlichungsnummer: **0 237 773**
**B1**

(12) # EUROPÄISCHE PATENTSCHRIFT

(45) Veröffentlichungstag der Patentschrift:
**02.11.89**

(51) Int. Cl.⁴: **A61M 5/16, A61M 5/14**

(21) Anmeldenummer: **87101859.4**

(22) Anmeldetag: **11.02.87**

(54) Infusionsgerät.

(30) Priorität: **19.02.86 DE 3605319**

(43) Veröffentlichungstag der Anmeldung:
**23.09.87 Patentblatt 87/39**

(45) Bekanntmachung des Hinweises auf die Patenterteilung:
**02.11.89 Patentblatt 89/44**

(84) Benannte Vertragsstaaten:
**AT DE ES FR GB IT SE**

(56) Entgegenhaltungen:
**EP-A- 0 018 817**
**EP-A- 0 154 163**
**DE-A- 2 022 573**
**DE-A- 3 108 848**
**DE-A- 3 408 572**
**GB-A- 1 264 711**
**US-A- 3 990 443**
**US-A- 4 244 364**

(73) Patentinhaber: **Pfrimmer-Viggo GmbH + Co. KG,
Langemarckplatz 3 Postfach 28 80, D-8520 Erlangen(DE)**

(72) Erfinder: **Iwatschenko, Peter, Bürgerholzweg 4,
D-8524 Neunkircken(DE)**

(74) Vertreter: **Wuesthoff, Franz, Dr.-Ing. et al, Patentanwälte
Wuesthoff -v. Pechmann-Behrens-Goetz
Schweigerstrasse 2, D-8000 München 90(DE)**

ACTORUM AG

## Beschreibung

Die Erfindung betrifft ein Infusionsgerät mit einer spritzgegossenen Tropfkammer, einem Tropfen-Sensor, wie zum Beispiel einer Lichtschranke oder einem piezoelektrischen Element, einer Auswerteschaltung für die Ausgangssignale des Tropfen-Sensors, einer Stromquelle und einer Anzeigeeinrichtung zum optischen Anzeigen eines Ausgangssignals der Auswerteschaltung.

Derartige Infusionsgeräte mit einem sogenannten Tropfenzähler sind beispielsweise aus dem DE-GM 84 32 849, dem DE-GM 85 16 066, der DE-OS 34 08 572, der DE-OS 31 08 848, der EP-OS 154 163 und der DE-OS 34 03 969 bekannt.

Bei den in den vorstehenden Druckschriften beschriebenen gattungsgemäßen Infusionsgeräten geht es darum, die Einstellung der Tropfrate bei der Schwerkraftinfusion zu vereinfachen. Die Bedienungsperson (Krankenschwester) braucht die momentane Tropfrate in der Tropfkammer nicht von Hand mittels einer Stopuhr zu messen und eine Regulierklemme entsprechend einzustellen, vielmehr wird die Tropfrate mittels eines Tropfen-Sensors, wie z.B. einer Lichtschranke oder einem piezoelektrischen Element, automatisch gemessen und der Bedienungsperson angezeigt.

Als nachteilig bei den bekannten Infusionsgeräten mit automatischer Messung der Tropfrate und deren optischer Anzeige müssen deren relativ hohe Herstellungskosten angesehen werden, weshalb die bekannten Meß- und Anzeigeeinrichtungen im Unterschied zu den Tropfkammern und Schläuchen nicht als sogenannte "Einweg-Artikel" (welche nach einmaligem Gebrauch weggeworfen werden können) angeboten wurden, sondern für den oftmaligen Gebrauch bestimmt sind.

Auch haben bekannte Infusionsgeräte mit einer elektronischen Messung der Tropfrate und deren Anzeige den Nachteil, daß sich die Bedienungsperson auf die angezeigte Tropfrate verlassen muß, ohne einen freien Einblick in das Geschehen innerhalb der Tropfkammer zu haben. Es ist aber höchst erwünscht, daß die Bedienungsperson trotz der automatischen Meß- und Anzeigeeinrichtungen bezüglich der Tropfrate zusätzlich noch einen freien Einblick in die Tropfkammer von möglichst vielen Seiten hat, so daß sie jederzeit mit einem Blick überprüfen kann, ob das angezeigte Meßergebnis plausibel ist.

Für den Vertrieb, die Bevorratung und Lagerhaltung von Infusionsgeräten, welche einen Massenartikel darstellen, ist es wichtig, daß das gesamte Gerät als eine kompakte Einheit gehandhabt werden kann. Dies ist dann nicht möglich, wenn die Tropfkammer, die Regulierklemme und der Schlauch einerseits als "Einweg-Artikel" und die Meß- und Anzeigeeinrichtungen für die Tropfrate andererseits für den mehrfachen Gebrauch (als oftmalig mit verschiedenen Tropfkammern, Schläuchen etc. zu verwendende Artikel) vorgesehen sind.

Der Erfindung liegt die Aufgabe zugrunde, ein Infusionsgerät der eingangs beschriebenen Art zu schaffen, welches als kompakte Einheit kostengünstig herstellbar ist und der Bedienungsperson neben der elektronischen Anzeige der Tropfrate auch eine direkte visuelle Überprüfung des Tropfzustandes in der Tropfkammer zu ermöglicht.

Erfindungsgemäß ist zur Lösung dieser Aufgabe vorgesehen, daß integral in oder an der Tropfkammer Ausnehmungen zur Aufnahme des Tropfen-Sensors, der Auswerteschaltung, der Stromquelle und der Anzeigeeinrichtung ausgeformt sind.

Anstatt zusätzlich neben der (wegwerfbaren) Tropfkammer ein Meß- und Anzeigegerät in einem gesonderten Gehäuse vorzusehen, schlägt die Erfindung vor, bereits die Tropfkammer beim Spritzguß derart auszuformen, daß alle für die Messung und Anzeige der Tropfrate erforderlichen Bauteile, wie der Tropfen-Sensor, die Stromquelle etc., direkt in Ausnehmungen im Kunststoff der Tropfkammer eingefügt werden können. Ein gesondertes (Metall-) Gehäuse zur Aufnahme der elektrischen und opto-elektrischen Bauteile ist nicht erforderlich.

Bei der Herstellung der Tropfkammer kann bereits berücksichtigt werden, daß dieselbe mit Meß- und Anzeigeeinrichtungen für die Tropfrate versehen werden soll, so daß ein kompaktes Infusionsgerät geschaffen werden kann, welches als eine Einheit einmalig bei einem Patienten gebraucht und sodann weggeworfen wird.

In einer bevorzugten Ausgestaltung der Erfindung ist vorgesehen, daß die Ausnehmungen zur Aufnahme der Stromquelle, der Auswerteschaltung und der Anzeigeeinrichtung im Oberteil der Tropfenkammer vorgesehen sind. Somit bleibt ein Großteil der Tropfenkammer und insbesondere deren Unterteil frei von praktisch allen Seiten einsehbar, so daß die Bedienungsperson die fallenden Tropfen und den Flüssigkeitsspiegel in der Tropfenkammer direkt sehen kann und das auf der Anzeigeeinrichtung angezeigte Meßergebnis grob überprüfen kann. Die Bedienungsperson und der Patient müssen sich nicht "blind" auf das Funktionieren der Meßeinrichtung verlassen.

Die Ausnehmungen zur Aufnahme der genannten Bauteile sind vorzugsweise in einer Wand-Verstärkung der Tropfenkammer vorgesehen, die integral ausgebildet ist und sich über einen relativ kleinen Teil-Abschnitt der Gesamtlänge der Tropfkammer erstreckt. Von der Verstärkung stehen beiderseits der Tropfenkammer diametral gegenüberliegend zwei Arme nach unten ab, an deren Enden in einer bevorzugten Weiterbildung der Erfindung der Sender und der Empfänger einer Lichtschranke angeordnet sind. Diese den Tropfen-Sensor bildende Lichtschranke deckt somit die Tropfkammer kaum ab und muß nicht gesondert montiert werden.

In einer bevorzugten Ausgestaltung der Erfindung ist als Stromquelle eine Solar-Zelle an der Tropfkammer vorgesehen.

Es versteht sich, daß bei einem erfindungsgemäßen Infusionsgerät die Stromversorgung über einen langen Zeitraum sichergestellt sein muß. Damit nicht unnötig Strom für den Betrieb des Tropfensensors, der Auswerteschaltung und/oder der Anzeigeeinrichtung verbraucht wird, wenn diese Bauteile nicht in ihrem bestimmungsgemäßen Betrieb sind, ist in einer bevorzugten Ausgestaltung der Erfindung vorgesehen, daß eine den Einstechdorn der Tropfkammer abdeckende Schutzkappe die elektrische Verbindung zwischen der Stromquelle und den Ver-brauchern unterbricht. Wenn die Bedienungsper-son das erfindungsgemäße Infusionsgerät aus seiner Verpackung auspackt und den Einstechdorn in einen Gummistopfen der Infusionsflasche sticht, nimmt sie zuvor die Schutzkappe ab, so daß erst mit dem tatsächlichen Betrieb des Infusionsgerätes auch der Anschluß der Stromquelle an die Meß- und Anzeigeeinrichtungen erfolgt.

Die elektrischen Leitungen zwischen der Stromquelle und den Verbrauchern sind in die Tropfkammer-Wand oder die Verstärkung eingelegt.

In einer bevorzugten Weiterbildung der Erfindung ist die an der Tropfkammer vorgesehene Meß-und Anzeigeeinrichtung, wie sie vorstehend beschrieben worden ist, mit einer Regulierklemme für die Durchflußrate der Infusionsflüssigkeit durch den Infusionsschlauch derart gekoppelt, daß ein vollständiger Regelkreis gebildet wird. Hierzu ist die Auswerteschaltung der Meßeinrichtung für die Tropfrate mit einem Antrieb einer Regulierklemme verbunden, wodurch die Durchflußrate durch den von der Regulierklemme beaufschlagten Schlauch entsprechend einem Ausgangssignal der Auswerteschaltung einstellbar ist.

Auch das um eine Regulierklemme ergänzte Infusionsgerät kann kostengünstig als eine Wegwerf-Einheit hergestellt werden.

Eine für die Regelung der Durchflußrate besonders geeignete Regulierklemme ist in den Unteransprüchen 8 bis 10 beschrieben.

Nachfolgend wird die Erfindung anhand eines in der Zeichnung dargestellten Ausführungsbeispieles näher erläutert. Es zeigt:

Fig. 1 schematisch eine Tropfkammer mit einer optischen Anzeige für die Tropfrate;
Fig. 2 die in Fig. 1 gezeigte Tropfkammer mit einer Schutzkappe;
Fig. 3 die in den Fig. I und 2 gezeigte Tropfkammer einschließlich einer Regulierklemme;
Fig. 4 einen Schnitt entlang der Linie I-II der Fig. 3;
Fig. 5 einen Schnitt entlang der Linie III-IV der Fig. 3;
Fig. 6 ein Block-Diagramm der Auswerteschaltung;
Fig. 7 einen Schnitt durch die Regulierklemme entlang der Linie V-VI der Fig. 3; und
Fig. 8 einen Schnitt entlang der Linie A-B der Fig. 7.

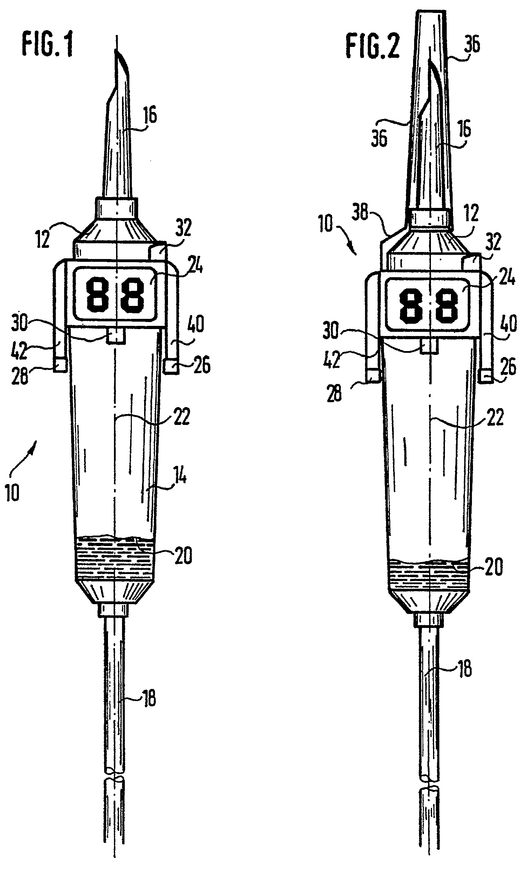

Gemäß Fig. 1 weist die Tropfkammer 10 ein Oberteil 12 und ein Unterteil 14 auf, welche koaxial ineinandergesteckt sind. Das Oberteil 12 der Tropfkammer ist mit einem Einstechdorn 16 versehen, welcher in den Gummistopfen (nicht gezeigt) einer Infusionsflasche (nicht gezeigt) einstechbar ist. Für die Schwerkraft-Infusion ist die Tropfkammer gemäß den Fig. 1 bis 3 vertikal ausgerichtet.

Von der Tropfkammer 10 führt ein Schlauch 18 zum Patienten (nicht gezeigt). In der Tropfkammer 10 bildet sich entsprechend der eingestellten Tropfrate ein Flüssigkeitsspiegel 20. Die Tropfen fallen aus dem Tropfenauslauf 30 entlang der Längsachse 22 der Tropfkammer nach unten auf den Flüssigkeitsspiegel 20.

Mittels einer optischen Anzeigeeinrichtung, wie einem LCD (Liquid Cristal Display) wird die gemessene Tropfrate der Bedienungsperson angezeigt.

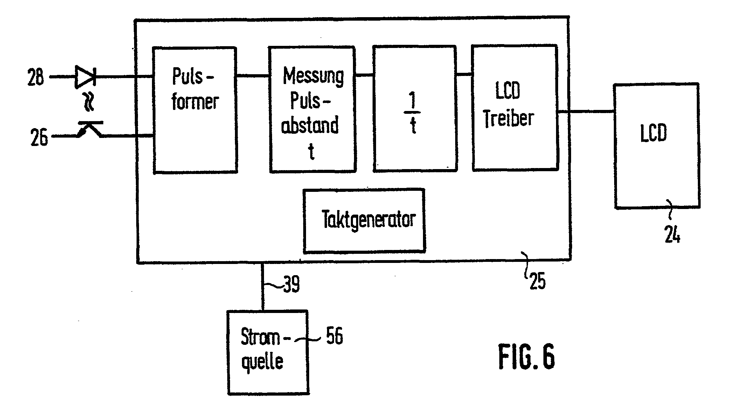

Zur Ermittlung der Tropfrate ist eine auf einem Halbleitersubstrat integrierte Auswerteschaltung 25 (Fig. 4 und 6) vorgesehen, welche weiter unten beschrieben wird. Zur Ermittlung der Tropfrate ist eine aus einem Licht-Sender 26 und einem Licht-Empfänger 28 bestehende Lichtschranke vor-gesehen, wobei die aus dem Tropfenauslauf 30 herabfallenden Tropfen die Lichtschranke passieren.

In den Fig. 1 und 2 ist mit dem Bezugszeichen 32 ein Ansatz für einen Luftfilter versehen, so daß der Innenraum der Tropfkammer 10 Atmosphärendruck aufweist.

Gemäß Fig. 2 ist der Einstechdorn 16 mit einer transparenten Schutzkappe 36 abgedeckt, welche an ihrem unteren Ende mit einer Isolierscheibe 38 versehen ist, die die Stromquelle (Fig. 4) von den Verbrauchern, d.h. insbesondere der Auswerteschaltung 25, der optischen Anzeigeeinrichtung 24 und der Lichtschranke aus Sender 26 und Empfänger 28 trennt. Erst wenn die Bedienungsperson die Schutzkappe 36 und die Isolierscheibe 38 entfernt hat, steht die Stromquelle mit den genannten Verbrauchern in elektrischer Verbindung (s. Leitung 39 in Fig. 6).

Der Sender 26 und der Empfänger 28 der Lichtschranke (Tropfen-Sensor) sind an zwei Armen 40, 42 angeordnet, die sich diametral gegenüberliegend parallel zur Längsachse 22 der Tropfenkammer 10 erstrecken.

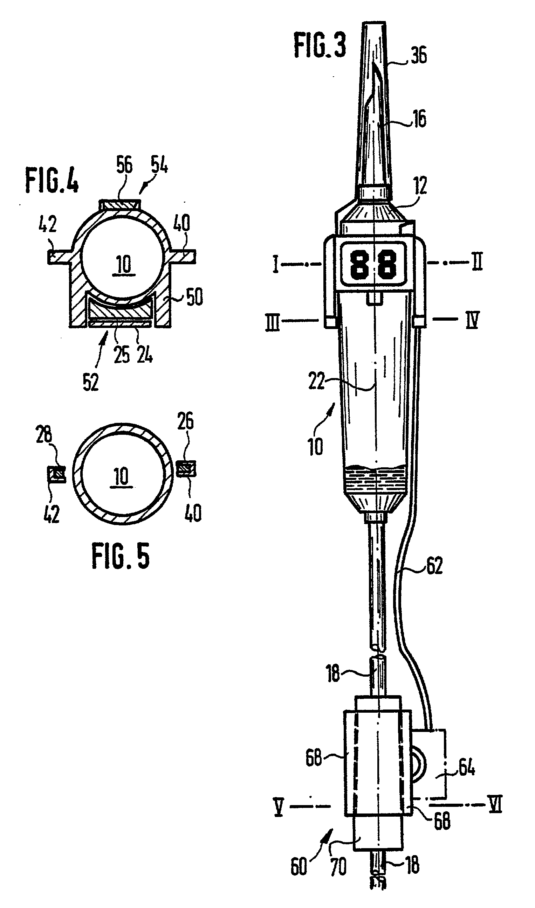

Gemäß Fig. 4, welche einen Schnitt entlang der Linie I-II der Fig. 3 zeigt, sind die optische Anzeigeeinrichtung 24 und die Auswerteschaltung 25 in einer Verstärkung 50 der Tropfenkammer 10 angeordnet. Die Verstärkung 50 weist eine Ausnehmung 54 für die Anzeigeeinrichtung 24 und die Auswerteschaltung 25 auf. Eine weitere Ausnehmung 54 ist für die Stromquelle 56 vorgesehen, welche z.B. eine Solar-Zelle sein kann.

Fig. 5 zeigt einen Schnitt entlang der Linie III-IV der Fig. 3 einschließlich der Tropfkammer 10 und der aus Sender 26 und Empfänger 28 bestehenden Lichtschranke.

Fig. 6 zeigt das Block-Schaltbild der Auswerteschaltung einschließlich der Stromquelle 56 und der optischen Anzeigeeinrichtung 24.

Die von der Lichtschranke (Sender 26, Empfänger 28) stammenden Signale werden in einen Pulsformer eingegeben, dessen Ausgangssignal in einen Meßkreis für den Puls-Abstand t gegeben wird. Der Abstand der Meß-Pulse entspricht dem Abstand aufeinanderfolgender Tropfen. Der reziproke Wert 1/t ist die Tropfrate. Entsprechend der berechneten Tropfrate wird ein LCD-Treiber beaufschlagt, welcher mit der optischen Anzeigeeinrichtung 24 verbunden ist.

Statt der Lichtschranke aus Sender 26 und Empfänger 28 kann auch ein an sich bekannter piezoelektrischer Kristall vorgesehen sein, dessen Pulse in den Pulsformer eingegeben werden.

Fig. 3 zeigt eine Regulierklemme 60, die über eine Leitung 62 mit der Auswerteschaltung 25 verbunden ist. Die Regulierklemme 60 steuert den Strömungsquerschnitt des Schlauches 18 entsprechend einem gewünschten Sollwert der Tropfrate. Dadurch können patientenseitige Druckschwankungen ausgeglichen werden, so daß die Infusionsgeschwindigkeit immer einen konstanten Sollwert annimmt.

Die Regulierklemme 60 ist mit einem Antrieb 64 (Motor) versehen, welcher über eine Leitung 62 mit der Auswerteschaltung 25 verbunden ist und aus dieser seine Steuersignale erhält.

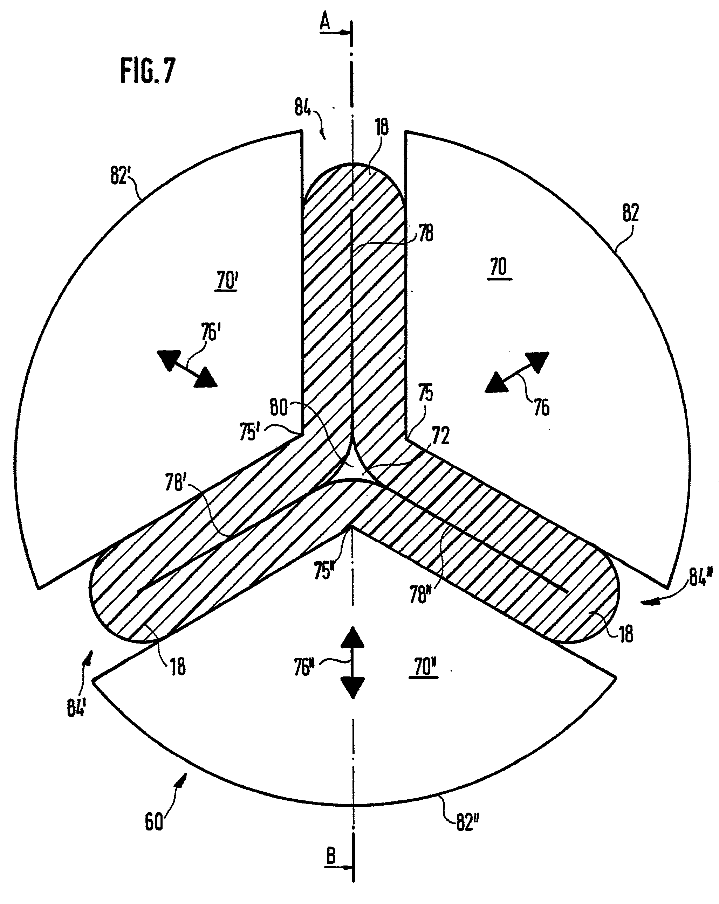

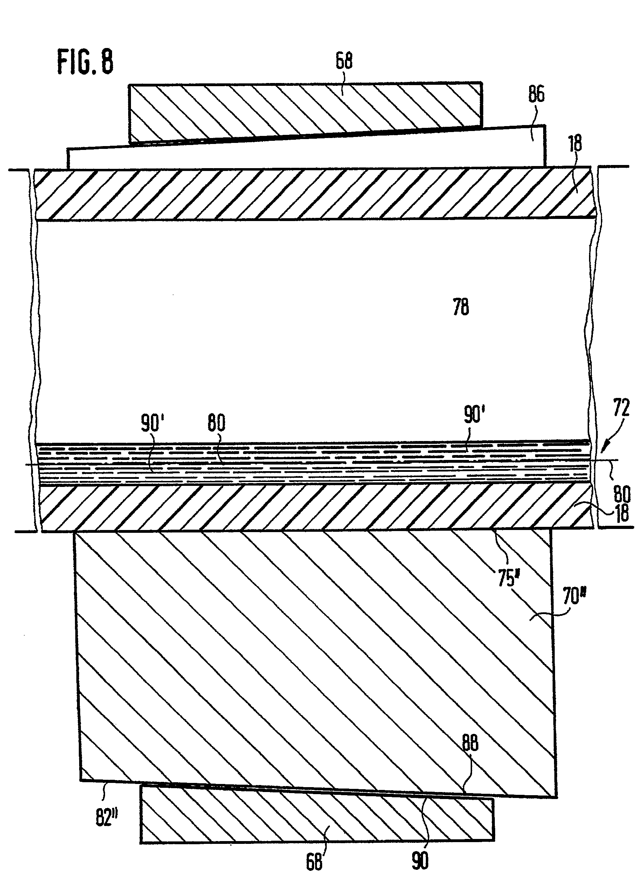

Die Fig. 7 und 8 zeigen Einzelheiten der Regulierklemme 60.

Gemäß Fig. 7 wird der Schlauch 18 aus PVC mittels der Klemmbacken 70, 70', 70" auf den Strömungsquerschnitt 72 verengt. Hierzu sind die Klemmbacken jeweils entlang der Radien 76, 76', 76" in Richtung der Pfeile verschiebbar. Die zentralen Kanten 75, 75', 75" der kreissektorförmigen Klemmbacken 70, 70', 70" bewegen sich auf den Radien 76, 76', 76", so daß der Schlauch 18 in einen um seine Längsachse 80 rotationssymmetrischen Klemmzustand überführt wird. Gemäß Fig. 7 liegen dabei die Innenwände des Schlauches 18 entlang der Anlageflächen 78, 78', 78" weitgehend planparallel dicht aneinander, so daß nur der im Schnitt dreieckförmige Strömungsquerschnitt 72 mit konkaven Seitenflächen freibleibt.

Die Umfangsabschnitte 82, 82', 82" der Klemmbacken 70, 70', 70" bilden zumindest annähernd einen Zylinder oder Konus. Zwischen den Klemmbacken bleiben gemäß Fig. 7 Freiräume 84, 84', 84", in denen der Schlauch 18 jeweils teilweise abgeklemmt ist.

Fig. 8 zeigt einen Schnitt entlang der Linie A-B der Fig. 7. Zusätzlich zu den in Fig. 7 gezeigten Bauteilen sind noch ein Zentriereinsatz 86 und eine äußere Hülse 68 vorgesehen. Der Zentriereinsatz 86 ist jeweils in den Freiräumen 84, 84', 84" zwischen den Klemmbacken vorgesehen und bewirkt, daß jeweils gleichlange Abschnitte des Schlauches 18 in den Freiräumen 84, 84', 84" abgeklemmt werden. Die Zentriereinsätze 86 schließen außenseitig bündig mit den Umfangsabschnitten 82, 82', 82" der Klemmbacken ab und ergänzen diese zumindest annähernd zu einem vollen Kreis (nicht gezeigt).

Auf den Zentriereinsatz kann insbesondere dann verzichtet werden, wenn der Schlauch 18 zumindest im Bereich der Klemmbacken 70, 70' und 70" derart vorgeformt ist, daß die Kanten 75, 75', 75" der Klemmbacken zwangsläufig rotationssymmetrisch mit einem Drehwinkel von 120° am Schlauch 18 angreifen. Hierzu ist der Schlauch 18 von vorneherein im Bereich der Regulierklemme im Schnitt etwa dreieckförmig plastisch vorgeformt und weist drei sich parallel zur Längsachse 80 erstreckende Führungsrillen (nicht gezeigt) auf, in welche die spitzen Kanten 75, 75', 75" der Klemmbacken derart eingreifen, daß in den Freiräumen 84, 84', 84" jeweils gleiche Schlauchabschnitte abgeklemmt werden.

Im in Fig. 8 gezeigten Ausführungsbeispiel werden die Klemmbacken 70, 70', 70" entlang der durch die Längsachse 80 des Schlauches 18 gehenden Radien 76, 76', 76" mittels einer Hülse 68 bewegt. Hierfür sind die äußeren Umfangsabschnitte 82, 82', 82" der Klemmbacken mit einem Außengewinde 88 und der Innenumfang der Hülse 68 mit einem Innengewinde 90 versehen. Je nach dem Schraubzustand zwischen Hülse und Klemmbacken wird somit der Strömungsquerschnitt 72 des Schlauches 18 verändert und die Fließgeschwindigkeit der Flüssigkeit 90' gesteuert.

Gemäß Fig. 8 erstrecken sich die spitzen Kanten 75, 75', 75" der Klemmbacken zumindest annähernd parallel zur Längsachse 80 des Schlauches 18. Somit kann ein relativ langer Schlauchabschnitt von z.B. 2 bis 5 cm abgeklemmt werden und die Drosselung der Fließgeschwindigkeit mittels der Regulierklemme weist eine gute Dämpfung auf, d.h. patientenseitige Druckschwankungen werden nur sehr stark gedämpft auf die andere Seite der Regulierklemme übertragen, so daß die Fließgeschwindigkeit bei momentanen patientenseitigen Druckschwankungen nicht sofort vom Sollwert abweicht.

Der Antrieb (Elektromotor 64) ist durch die Stromquelle 56 versorgt und wird durch die Auswerteschaltung (25) derart gesteuert, daß die Tropfrate einen vorgegebenen Sollwert einnimmt. Der Antrieb 64 dreht somit die Hülse 68 der Regulierklemme 60 derart, daß der Strömungsquerschnitt 72 so geändert wird, daß der Sollwert der Tropfrate erreicht wird.

Hierzu stellt die Bedienungsperson (nach Entfernung der Schutzkappe 36 und dem Einstechen des Einstechdornes 16 in die Infusionsflasche) zunächst einen Sollwert ein, der mittels der optischen Anzeigeeinrichtung 24 angezeigt wird. Die Auswerteschaltung 25 ist so ausgelegt, daß z.B. in den ersten fünf Minuten der Stromversorgung (also nach dem Entfernen der Schutzkappe 36) keine Regelung erfolgt, so daß die Bedienungsperson genügend Zeit hat, um den Sollwert einzustellen. Erst nach Ablauf der vorgegebenen Zeitspanne von z.B. fünf Minuten setzt die Regelung der Durchflußrate mittels der Regulierklemme 60 ein, und zwar auf ± 20 % des Sollwertes. Werden diese Grenzwerte überschritten, so gibt die Anzeigeeinrichtung 24 Alarm, beispielsweise durch Blinken des angezeigten Signales. Auch kann eine gesonderte Schallquelle für den Alarm vorgesehen sein.

Das gesamte Infusionsgerät bestehend aus der Tropfkammer 10 einschließlich der Meß- und Anzeigeeinrichtungen mit Sender 26, Empfänger 28, Auswerteschaltung 25 und Anzeigeeinrichtung 24, sowie die Regulierklemme 60 einschließlich des Motors 64 bilden eine Einheit, die eine vollautomatische Anzeige und Regelung der Tropfrate mit einem Ausgleich von patientenseitigen Druckschwankungen ermöglicht.

**Patentansprüche**

1. Infusionsgerät mit einer spritzgegossenen Tropfkammer (10), einem Tropfen-Sensor, wie zum Beispiel einer Lichtschranke (26, 28) oder einem piezoelektrischen Element, einer Auswerteschaltung (25) für die Ausgangssignale des Tropfen-Sensors, einer Stromquelle (56) und einer Anzeigeeinrichtung (24) zum optischen Anzeigen eines Ausgangssignals der Auswerteschaltung, dadurch **gekennzeichnet,** daß integral in oder an der Tropfkammer (10) Ausnehmungen (52, 54) zur Aufnahme zumindest des Tropfen-Sensors (26, 28), der Auswerteschaltung (25), der Stromquelle (56) und der Anzeigeeinrichtung (24) ausgeformt sind.

2. Infusionsgerät nach Anspruch 1, dadurch **gekennzeichnet,** daß die Ausnehmungen (52, 54) in dem Oberteil (12) der Tropfkammer (10) ausgeformt sind.

3. Infusionsgerät nach einem der Ansprüche 1 oder 2, dadurch **gekennzeichnet,** daß die Tropfkammer (10) bzw. deren Oberteil (12) eine integral ausgeformte Verstärkung (50) aufweist, die sich über einen relativ kleinen Abschnitt der Gesamtlänge der Tropfkammer (10) erstreckt und die Ausnehmungen (52, 54) für die Stromquelle (56), die Auswerteschaltung (25) und die Anzeigeeinrichtung (24) aufweist.

4. Infusionsgerät nach Anspruch 3, dadurch **gekennzeichnet,** daß die Verstärkung (50) beiderseits der Tropfkammer (10) diametral gegenüberliegend zwei Arme (40, 42) aufweist, die parallel zur Längsachse (22) der Tropfkammer (10) in deren Betriebsstellung nach unten abstehen und daß an den Enden der Arme (40, 42) Ausnehmungen für den Sender (26) und Empfänger (28) einer Lichtschranke als Tropfen-Sensor vorgesehen sind.

5. Infusionsgerät nach einem der vorhergehenden Ansprüche, dadurch **gekennzeichnet,** daß als Stromquelle (56) eine Solar-Zelle vorgesehen ist.

6. Infusionsgerät nach einem der Ansprüche 1 bis 4, dadurch **gekennzeichnet,** daß als Stromquelle (56) eine Batterie vorgesehen ist.

7. Infusionsgerät nach einem der vorhergehenden Ansprüche mit einem Einstechdorn (16) am Oberteil (12) der Tropfkammer (10), dadurch **gekennzeichnet,** daß eine Schutzkappe (36) für den Einstechdorn (16) die elektrische Verbindung (39) zwischen der Stromquelle (56) und der Auswerteschaltung (25) sowie der Anzeigeeinrichtung (24) unterbricht.

8. Infusionsgerät nach einem der vorhergehenden Ansprüche, dadurch **gekennzeichnet,** daß die Auswerteschaltung (25) mit einem Antrieb (64) für eine Regulierklemme (60) verbunden ist und daß die Durchflußrate durch den von der Regulierklemme (60) beaufschlagten Schlauch (18) entsprechend einem Ausgangssignal der Auswerteschaltung (25) steuerbar ist.

9. Infusionsgerät nach Anspruch 8, dadurch **gekennzeichnet,** daß die Regulierklemme (60) zum Ändern des Strömungsquerschnittes (72) des Schlauches (18) zumindest drei den Schlauch (18) zwischen sich einklemmende Klemmbacken (70, 70', 70") aufweist, deren relativer Abstand veränderbar ist.

10. Infusionsgerät nach einem der Ansprüche 8 oder 9, dadurch **gekennzeichnet,** daß die Klemmbacken (70, 70', 70") im Schnitt senkrecht zur Längsachse (80) des Schlauches (18) zumindest annähernd kreissektorförmig sind.

11. Infusionsgerät nach einem der Ansprüche 8 bis 10, dadurch **gekennzeichnet,** daß die Klemmbacken (70, 70', 70") entlang von Radien (76, 76', 76") mittels einer drehenden Hülse (68) verschiebbar sind, wobei die drehende Hülse (68) vom Antrieb (64) beaufschlagt ist.

**Claims**

1. An infusion device comprising an injection molded drip chamber (10), a drop sensor, such as a light barrier (26, 28) or a piezoelectric member, an evaluation circuit (25) for the output signals of the drop sensor, a power source (56), and a display means (24) for optical display of an output signal provided by the evaluation circuit, characterized in that recesses (52, 54) are formed integrally in or at the drip chamber (10) to receive at least the drop sensor (26, 28), the evaluation circuit (25), the power source (56), and the display means (24).

2. The infusion device as claimed in claim 1, characterized in that the recesses (52, 54) are formed in the upper part (12) of the drip chamber (10).

3. The infusion device as claimed in claim 1 or 2, characterized in that the drip chamber (10) or its upper part (12) includes an integrally formed reinforcement (50) which extends for a relatively small portion of the total length of the drip chamber (10) and includes the recesses (52, 54) for the power source (56), the evaluation circuit (25), and the display means (24).

4. The infusion device as claimed in claim 3, characterized in that the reinforcement (50) comprises two arms (40, 42) which are diametrically opposed at either side of the drip chamber (10) and project downwardly in parallel with the longitudinal axis (22) of the drip chamber (10) in the operative position of the same, and in that the ends of the arms (40, 42) are formed with recesses for the transmitter (26) and receiver (28) of a light barrier serving as drop sensor.

5. The infusion device as claimed in one of the preceding claims, characterized in that a solar cell is provided as the power source (56).

6. The infusion device as claimed in one of claims 1 to 4, characterized in that a battery is provided as the power source (56).

7. The infusion device as claimed in one of preceding claims, comprising a piercing tip (16) at the upper part (12) of the drip chamber (10), characterized in that a protective cap (36) for the piercing tip (16) interrupts the electrical connection (39) between the power source (56) and the evaluation circuit (25) as well as the display means (24).

8. The infusion device as claimed in one of the preceding claims, characterized in that the evaluation circuit (25) is connected to a drive means (64) for a control clamp (60), and in that the rate of flow through the hose (18) acted upon by the control clamp (60) is controllable by an output signal of the evaluation circuit (25).

9. The infusion device as claimed in claim 8, characterized in that the control clamp (60) for varying the flow cross section (72) of the hose (18) comprises at least three clamping jaws (70, 70`, 70``) between which the hose (18) is clamped and the relative spacing of which is variable.

10. The infusion device as claimed in claim 8 or 9, characterized in that clamping jaws (70, 70`, 70``) are at least approximately of circular sector shape in a section perpendicular to the longitudinal axis (80) of the hose (18).

11. The infusion device as claimed in one of claims 8 to 10, characterized in that the clamping jaws (70, 70`, 70``) are displaceable along radii (76, 76`, 76``) by means of a rotating sleeve (68) which is acted upon by the drive means (64).

**Revendications**

1. Dispositif de perfusion comprenant une chambre de goutte-à-goutte (10) moulée par injection, un détecteur de gouttes tel que, par exemple, un détecteur photo-électrique (26, 28) ou un élément piézo-électrique, un circuit d'analyse (25) pour les signaux de sortie du détecteur de gouttes, une source de courant (56) et un dispositif d'affichage (24) pour l'indication optique d'un signal de sortie du circuit d'analyse, caractérisé en ce que des évidements (52, 54) destinés à loger au moins le détecteur de gouttes (26, 28), le circuit d'analyse (25), la source de courant (56) et le dispositif d'affichage (24) sont venus de moulage dans ou sur la chambre de goutte-à-goutte (10).

2. Dispositif de perfusion selon la revendication 1, caractérisé en ce que les évidements (52, 54) sont venus de moulage dans la partie supérieure (12) de la chambre de goutte-à-goutte (10).

3. Dispositif de perfusion selon une des revendications 1 et 2, caractérisé en ce que la chambre de goutte-à-goutte (10) ou sa partie supérieure (12) présente un renforcement unitaire venu de moulage (50) qui s'étend sur un segment relativement petit de la longueur totale de la chambre de goutte-à-goutte (10) et présente les évidements (52, 54) destinés à recevoir la source de courant (56), le circuit d'analyse (25) et le dispositif indicateur (24).

4. Dispositif de perfusion selon la revendication 3, caractérisé en ce que le renforcement (50) présente, de part et d'autre de la chambre de goutte-à-goutte (10), deux bras (40, 42) qui sont diamétralement opposés l'un à l'autre, qui font saillie vers le bas parallèlement à l'axe longitudinal (22) de la chambre de goutte-à-goutte (10) dans leur position de travail, et en ce qu'aux extrémités des bras (40, 42) sont prévus des évidements qui reçoivent l'émetteur (26) et le récepteur (28) d'un détecteur photo-électrique servant de détecteur de gouttes.

5. Dispositif de perfusion selon une des revendications précédentes, caractérisé en ce qu'une pile solaire est prévue en qualité de source de courant (56).

6. Dispositif de perfusion selon une des revendications 1 à 4, caractérisé en ce qu'une pile est prévue en qualité de source de courant.

7. Dispositif de perfusion selon une des revendications précédentes, comprenant une aiguille (16) sur la partie supérieure (12) de la chambre de goutte-à-goutte (10), caractérisé en ce qu'un capuchon protecteur (36) protégeant l'aiguille (16) interrompt la connexion électrique (39) entre la source de courant (56) et le circuit d'analyse (25) et le dispositif d'affichage (24).

8. Dispositif de perfusion selon une des revendications précédentes, caractérisé en ce que le circuit d'analyse (25) est relié à un entraînement (64) pour une pince de réglage (60) et en ce que le débit d'écoulement peut être commandé par le tuyau (18) serré par la pince de réglage (60) en fonction du signal de sortie du circuit d'analyse (25).

9. Dispositif de perfusion selon la revendication 8, caractérisé en ce que la pince de réglage (60) présente, pour modifier la section d'écoulement (72) du tuyau (18), au moins trois mâchoires de pince (70, 70`, 70``) qui serrent entre elles le tuyau (18), et dont l'écartement mutuel est variable.

10. Dispositif de perfusion selon une des revendications 8 et 9, caractérisé en ce que les mâchoires (70, 70`, 70``) de la pince sont de forme au moins approximativement en secteur de cercle dans une section perpendiculaire à l'axe longitudinal (80) du tuyau (18).

11. Dispositif de perfusion selon une des revendications 8 à 10, caractérisé en ce que les mâchoires (70, 70`, 70``) peuvent se déplacer en translation le long de rayons (76, 76', 76``), sous l'action d'une douille tournante (68), la douille tournante (68) étant attaquée par l'entraînement (64).

FIG.1

FIG.2

EP 0 237 773 B1

FIG.3

FIG.4

FIG.5

28 ⊳

26

| Puls-<br>former | Messung<br>Puls-<br>abstand<br>t | $\frac{1}{t}$ | LCD<br>Treiber |
|---|---|---|---|

Taktgenerator

LCD

24

39

25

Strom-<br>quelle    56

FIG. 6

FIG. 7

FIG. 8